# EUROPÄISCHE PATENTANMELDUNG

(11) **EP 2 606 825 A1**
(43) Veröffentlichungstag der Anmeldung: **26.06.2013**
(21) Anmeldenummer: 12198715.0
(22) Anmeldetag: 20.12.2012
(51) Int. Cl.: A61B 6/00, A61B 19/00, A61B 6/12

(54) **Bilderzeugungsapparat und -methode zur Nuklearbildgebung**

(30) Priorität: 20.12.2011 DE 102011121708
(71) Anmelder: SurgicEye GmbH, 81671 München (DE)
(72) Erfinder: Wendler, Thomas, 81677 München (DE)
(74) Vertreter: Zimmermann & Partner

(57) **Zusammenfassung**

Ein System zur endoskopischen nuklearen Bilderzeugung wird bereitgestellt. Es umfasst eine bewegliche endoskopische Nuklearsonde, welche einen Kollimator und einen Detektor zur Detektion von radioaktiver Strahlung umfasst, wobei die endoskopische Nuklearsonde einen länglichen Körper mit einer Längsachse (X) aufweist, der ausgelegt ist in den Körper eines Patienten eingeführt zu werden, wobei der Detektor und der Kollimator am länglichen Körper der endoskopischen Nuklearsonde angeordnet ist, und wobei der Kollimator ein Sichtfeld mit einer Achse (Y) aufweist, wobei die Achse (Y) des Sichtfelds des Kollimators in Bezug auf die Achse (X) des länglichen Körpers der endoskopischen Nuklearsonde abgewinkelt ist; Mittel zum Bestimmen der Position und Orientierung des länglichen Körpers der endoskopischen Nuklearsonde; eine Auswertungseinheit, welche umfasst: ein Schnittstellensystem, einen Datenspeicherabschnitt zum Speichern der Informationen; Mittel zum Synchronisieren der Information über die Position und Orientierung des länglichen Körpers der endoskopischen Nuklearsonde mit der Information über die von der endoskopischen Nuklearsonde detektierte Strahlung; Mittel zur rechnerischen Bestimmung einer Ist-Position und Ist-Orientierung des länglichen Körpers der endoskopischen Nuklearsonde; einen Programmspeicherabschnitt, sowie Mittel zum Berechnen mindestens eines Qualitätsparameters des Bilds. Außerdem werden eine Nuklearsonde und ein Verfahren bereitgestellt.

## Beschreibung

Die vorliegende Erfindung bezieht sich auf eine Vorrichtung zur endoskopischen Bilderzeugung und Methoden zur Bilderzeugung mit einer Vorrichtung zur endoskopischen Bilderzeugung, speziell auf ein entsprechendes System mit verbesserter Bildgebung.

### HINTERGRUND

Qualitativ hochwertige Bilderzeugung ist von großem Interesse für einen weiten Bereich von Anwendungen. Insbesondere im medizinischen Bereich, wo die Gesundheit eines Patienten davon abhängen kann, ist eine bestmögliche Bilderzeugung beispielsweise als Basis für Operationen am Patienten erforderlich.

Für gewöhnlich werden medizinische Bilder entweder präoperativ erzeugt, wie etwa durch Computertomographie (CT), Kernspintomographie (NMR, MR, MRI, MRT), Positronen-Emissions-Tomographie (PET), Einzelphotonen-Emissions-Tomographie (SPECT), Ultraschall (US) - oder andererseits intraoperativ (io) erzeugt, wie etwa durch io CT, io MRI, io US, oder Freihand SPECT. Auch ein Registrieren von Bildern ist bekannt, beispielsweise das Registrieren eines anatomischen Bildes (wie etwa eines CT-, MRI- oder US-Bilds) mit einem funktionellen Bild (wie etwa einem PET-oder SPECTBild), d.h. einem Bild, das die örtliche Verteilung einer Funktion bzw. einer Körperaktivität sichtbar macht. Solche registrierten Bilder können beispielsweise bei Tumoroperationen helfen zu entscheiden, welche Gewebeteile aus der anatomischen und funktionellen Information herauszuschneiden sind. Wünschenswert sind möglichst aktuelle und hochwertige Bilder, da so vermieden werden kann, gesundes Gewebe zu schädigen oder krankes aus Versehen nicht zu entfernen.

Hochwertige Bilder zu erzeugen stellt hohe Anforderungen an Detektordaten zur Bilderzeugung und an ein Auswertesystem, das diese Daten verarbeiten muss. Das gilt besonders für die Verarbeitung von Detektordaten mit beweglichen Detektoren, die beispielsweise in der Hand getragen/geführt werden und/oder sich innerhalb einer Kavität oder einer Lumina des Körpers befinden.

Der Stand der Technik im Zusammenhang mit der vorliegenden Erfindung ist beispielsweise in US 6,602,488, US 6,456,869, US 6,317,622 oder US 6,167,296 offenbart und ermöglicht das Tracking (die Nachverfolgung) der handgehaltenen Sonden als übliche Diagnosegeräte, insbesondere während eines chirurgischen Eingriffs, sowie Trackingsysteme (Nachverfolgungssysteme) zur Bestimmung der Position und Orientierung von Operationsinstrumenten und Bildgebungsgeräten.

Die Idee des Trackings von Nuklearsonden wurde in der Vergangenheit schon von mehreren Gruppen erwähnt, beispielsweise wie in US 6,510,336 und US 6,021,341 offenbart. Wie weiterhin in US 6,643,538 offenbart, können Nuklearsonden konstruktionsgemäß mit einer Kamera integriert werden.

Das Ausgabesignal von Nuklearsonden ist in der Regel lediglich ein eindimensionales Signal, das zeitlich nicht konstant ist. Die Hauptvorteile solcher Vorrichtungen sind die Tragbarkeit, Einfachheit, und die Möglichkeit ihrer Miniaturisierung zur Untersuchung von Hohlräumen, beispielsweise wenn auf Endoskopen montiert. Da jede Messung darüber hinaus nicht auf eine bestimmte Position bezüglich der vorherigen beschränkt ist, ermöglichen Sonden des Weiteren die Abtastung beliebiger Oberflächen mit einer räumlichen Genauigkeit, die nur durch die Größe des Sensors und die Reichweite der detektierten Nuklearstrahlung begrenzt wird.

Nuklearsonden, wie z. B. Gamma- und Beta-Sonden, können den radioaktiven Zerfall von Radionukliden in Tracern messen, die dem Patienten vor dem Eingriff injiziert werden. Der Nachteil dieser Nuklearsonden ist die Tatsache, dass es sich nur um Punktmessungen handelt. Dies erschwert die Wägung des physikalischen Werts auf einer Oberfläche, wenn er sich mit der Position erheblich verändert. Ein weiteres Problem dabei ist die Schwankung der Messergebnisse, welche auf der statistischen Natur des Zerfalls- und Detektionsprozesses basiert, was die Interpretation der Messdaten schwierig und potenziell unzuverlässig macht.

Die Verwendung derartiger Sonden zur Kombination von Positions- und Orientierungstracking mit Oberflächenrekonstruktion und Visualisierung wurde in der WO 2007/131561 A2 beschrieben. Dieses Verfahren ist als Freihand-SPECT bekannt und wird bereits bei offen-chirurgischen Eingriffen, d.h. bei geöffnetem Körper des Patienten eingesetzt, dagegen nicht im minimal-invasiven Bereich. Es beinhaltet unter anderem eine Messung der Emission des Zielgewebes durch eine Nuklearsonde mit im Wesentlichen in deren Längsrichtung gebündelter Detektionscharakteristik, sowie einen Abgleich dieser Daten mit Positionsinformationen über die Sonde selbst.

Ein Nachteil aller oben genannten Verfahren ist, dass die im allgemeinen handgeführte Nuklearsonde die Daten für ein daraus berechnetes Bild liefert, und dass z.B. durch eine zufällig ungünstige Führung der Sonde durch den Nutzer die Qualität des aus den detektierten Daten erzeugten Bildes nicht optimal sein kann, bzw. verbesserungsfähig sein kann. Dies kann vor allem bei der Führung von endoskopischen Nuklearsonden, wo die Bewegungsmöglichkeiten, unter anderem wegen der Durchführung durch die Haut oder durch Körperöffnungen, sehr beschränkt sind, sehr schwierig sein.

Vor diesem Hintergund ist die Aufgabe der vorliegenden Erfindung, ein System und Verfahren zur intrakorporalen Bildgebung bereitzustellen, insbesondere bei computergeführten Operation mit Nuklearsonden, das gegenüber bekannten Verfahren eine verbesserte Bildqualität liefert.

ZUSAMMENFASSUNG

Die oben angesprochenen Probleme werden zumindest teilweise gelöst durch ein System zur endoskopischen nuklearen Bilderzeugung gemäß Anspruch 1, eine endoskopische Nuklearsonde gemäß Anspruch 12 und ein Verfahren gemäß Anspruch 13.

In einem Ausführungsbeispiel wird ein System zur endoskopischen nuklearen Bilderzeugung vorgeschlagen. Das System umfasst eine bewegliche endoskopische Nuklearsonde, welche einen Kollimator und einen Detektor zur Detektion von radioaktiver Strahlung umfasst, wobei die endoskopische Nuklearsonde einen länglichen Körper mit einer Längsachse (X) aufweist, der ausgelegt ist in den Körper eines Patienten eingeführt zu werden, wobei der Detektor und der Kollimator am länglichen Körper der endoskopischen Nuklearsonde angeordnet ist, und wobei der Kollimator ein Sichtfeld mit einer Achse (Y) aufweist, wobei die Achse (Y) des Sichtfelds des Kollimators in Bezug auf die Achse (X) des länglichen Körpers der endoskopischen Nuklearsonde abgewinkelt ist. Es umfasst weiter Mittel zum Bestimmen der Position und Orientierung des länglichen Körpers der endoskopischen Nuklearsonde; und eine Auswertungseinheit, welche umfasst: ein Schnittstellensystem zur Übermittlung von Daten mit Information über die detektierte radioaktive Strahlung, von Information über die Position und Orientierung des länglichen Körpers der endoskopischen Nuklearsonde an die Auswertungseinheit; einen Datenspeicherabschnitt zum Speichern der Informationen; Mittel zum Synchronisieren der Information über die Position und Orientierung des länglichen Körpers der endoskopischen Nuklearsonde mit der Information über die von der endoskopischen Nuklearsonde detektierte Strahlung; Mittel zur rechnerischen Bestimmung einer Ist-Position und Ist-Orientierung des länglichen Körpers der endoskopischen Nuklearsonde; einen Programmspeicherabschnitt mit einem Programm zum ggf. wiederholten Ermitteln mindestens eines Qualitätswerts hinsichtlich der Bilderzeugung aus den genannten Informationen.

In einem weiteren Ausführungsbeispiel wird eine endoskopische Nuklearsonde bereitgestellt. Die Sonde umfasst einen länglichen Grundkörper, einen Kollimator, einen Detektor zur Detektion von radioaktiver Strahlung, wobei die endoskopische Nuklearsonde eingerichtet ist, mit dem länglichen Grundkörper in den Körper eines Patienten eingeführt zu werden, und wobei der Kollimator an einem Ende angeordnet ist, und wobei der Kollimator ein Sichtfeld mit einer Achse (Y) aufweist, wobei die Achse (Y) des Sichtfelds des Kollimators in Bezug auf die Achse (X) des länglichen Körpers der endoskopischen Nuklearsonde abgewinkelt ist, so dass der Detektionsbereich der Sonde für radioaktive Strahlung in einem Winkelbereich von 0 Grad bis 180 Grad zur Längsachse liegt, und wobei die Detektionscharakteristik anisotrop in Bezug auf eine Rotationssymmetrie um die Längsachse der Sonde ist.

In einem weiteren Ausführungsbeispiel wird ein Verfahren zur endoskopischen nuklearen Bilderzeugung vorgeschlagen. Das Verfahren umfasst Einführen einer Nuklearsonde, die eine nicht-rotationssymmetrische Detektionscharakteristik aufweist, in den Körper eines Patienten; Aufnehmen von Strahlungsdaten mittels der Nuklearsonde; Aufnehmen von Positions- und Orientierungsdaten der Nuklearsonde, Berechnen eines dreidimensionalen Bildes unter Nutzung der vorgenannten Information; und Berechnen mindestens eines Qualitätswertes bzw. -parameters für das errechnete dreidimensionale Bild.

Weitere Merkmale, Aspekte und Details, die mit hier beschriebenen Ausführungsformen kombiniert werden können, werden in den abhängigen Ansprüchen, der Beschreibung und den Abbildungen offenbart.

### KURZBESCHREIBUNG DER ABBILDUNGEN

Damit die zuvor aufgeführten Merkmale im Detail besser verstanden werden können, wird eine speziellere Beschreibung mit Bezug auf Ausführungsformen der Erfindung gegeben. Die beigefügten Abbildungen beziehen sich auf Ausführungsformen der Erfindung und werden im folgenden kurz beschrieben:

Figur 1 zeigt ein System gemäß Ausführungsformen;

Figur 2 zeigt schematisch verschiedene Ausrichtungen einer Nuklearsonde gemäß Ausführungsbeispielen;

Figur 3 zeigt eine Darstellung von Soll-Positions- und Lagedaten auf einem Display gemäß Ausführungsbeispielen.

Figur 4 zeigt die Anwendung eines Systems gemäß Ausführungsformen;

Figur 5 zeigt ein weiteres System gemäß Ausführungsformen;

Figur 6 zeigt ein weiteres System gemäß Ausführungsformen;

Figur 7 zeigt ein Transformationsverfahren gemäß Ausführungsformen;

Figur 8 zeigt eine Nuklearsonde gemäß Ausführungsformen;

Figur 9 zeigt ein Transformationsverfahren gemäß Ausführungsformen.

Figur 10 zeigt ein bildgebendes Verfahren gemäß Ausführungsformen.

### AUSFÜHRLICHE BESCHREIBUNG

Die hier beschriebenen Verfahren benutzen Computersoftware zur Berechnung von verschiedenen Arten von Bildinformationen aus detektierten Informationen. Die dabei eingesetzten Verfahren und Algorithmen sind dem Fachmann entweder bekannt oder können ohne weiteres unter Einsatz seines Standard-Fachwissens auf Basis der hierin gemachten Angaben geschrieben werden. Sie werden daher nicht im Detail behandelt.

Im Folgenden werden die Begriffe "Virtual Reality" (VR) und "Augmented Reality" (AR) benutzt. Unter Augmented Reality (auch: erweiterte Realität) versteht man die computergestützte Erweiterung der Realitätswahrnehmung. Hierunter ist die Ergänzung von Bildern oder Videos (eines Patientenkörpers) mit computergenerierten Zusatzinformationen oder virtuellen Objekten mittels Einblendung/Überlagerung zu verstehen, hier also vor allem den aus den Nuklearsondendaten berechneten Bildinformationen, z.B. eines radioaktiv markierten Tumors. Virtual Reality ist dagegen die Darstellung der Wirklichkeit und ihrer physikalischen Eigenschaften in einer in Echtzeit computergenerierten virtuellen Umgebung. D.h. VR arbeitet im allgemeinen nur mit "vollsynthetisch" generierten/errechneten Daten, und nicht mit Bildern aus einem bildgebenden Verfahren, die AR mischt dagegen beides, Echt-Bilder und computergenerierte Bilder (auch "gemischte Realität" oder "mixed reality" genannt). Der Begriff "Pose" ist allgemein bekannt als sowohl die (3D-)Position eines Objekts im Raum angebend, als auch dessen Orientierung bzw. Lage. Der Begriff "Qualitätswert" oder "Qualitätsparameter", hier allgemein mit Q bezeichnet, repräsentiert einen im Kontext dieser Anmeldung verwendeten Parameter, der geeignet ist, die Qualität der hierin behandelten Bild-Darstellungen, Repräsentationen etc. zu repräsentieren und in rechnergestützten Verfahren verarbeitet zu werden. Q kann dabei, je nach gewähltem zugrundeliegenden Verfahren, auf den verschiedensten mathematischen Größen beruhen, wie unten näher beschrieben wird, von (nicht-limitierende Aufzählung) Skalaren über Vektoren bis hin zu beliebig-dimenisonalen Matrizen, Vektorfeldern oder Kombinationen der vorgenannten. Der hier verwendete Begriff "länglicher Körper" im Zusammenhang mit der Nuklearsonde bezeichnet mindestens einen Teilbereich der Sonde und bezieht sich auf die heute praktisch durchgängige Bauart solcher Sonden für den medizinischen Bereich, die einen stabförmigen Aufbau haben, mit dem Kollimator an einem Ende. Dies ist aber nicht als Einschränkung zu sehen; vielmehr kann eine Nuklearsonde, wie sie in Ausführungsbeispielen vorgeschlagen wird, durchaus auch als nicht-längliche Bauform realisiert sein, bei sonst gleicher oder ähnlicher Funktionalität, was daher als ebenfalls als im Sinne der Erfindung liegend begriffen wird, auch wenn die äußere Form im Einzelfall nicht direkt als länglich zu bezeichnen ist. Ferner sind die hierin beschriebenen Trackingsysteme, Sonden, Endoskope, etc. über Schnittstellen mit mindestens je einer Auswertungseinheit, Ausgabeeinheit und/oder miteinander verbunden. Diese aus dem Stand der Technik bekannten Schnittstellen können über kabellose Funksysteme oder per Kabel verbunden sein, wie aus dem Stand der Technik bekannt. Ferner sind hier genannte Mittel zum Zweck der Berechnung von 3D-Bildern, von Transformationen, Mittel zur Synchronisierung, etc., im allgemeinen als handelsübliche Rechnersysteme (Personal Computer bzw. Workstation) realisiert, auf denen entsprechend den Ausführungsbeispielen Algorithmen in Form von Computerprogrammen realisiert sind.

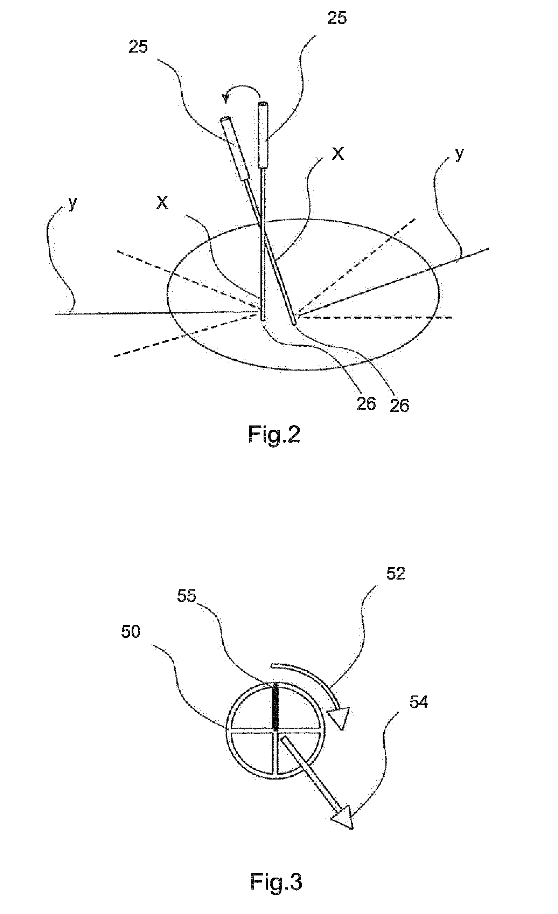

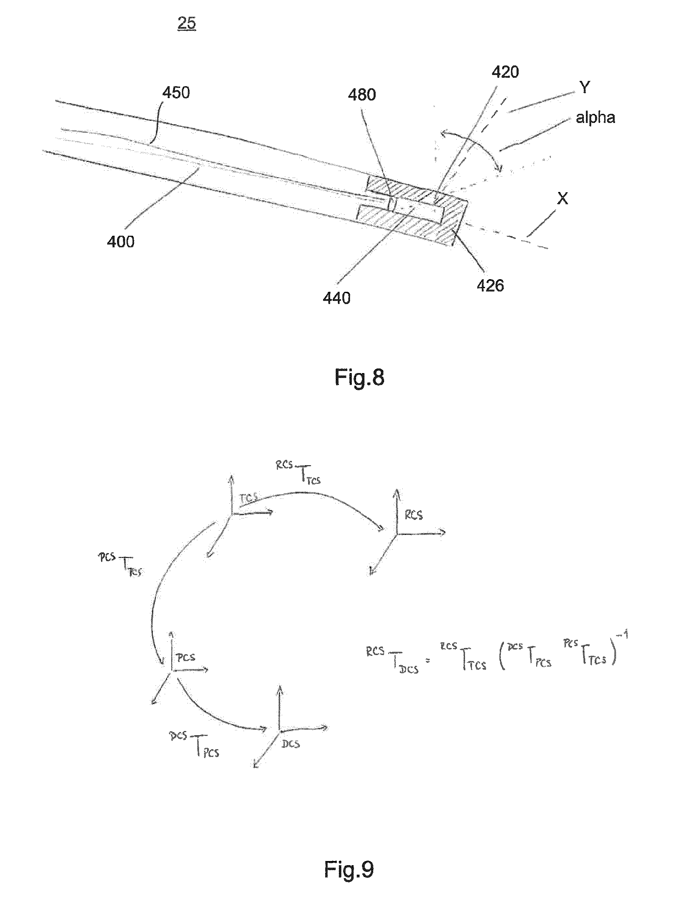

Ausführungsbeispiele betreffen ein System zur nuklearen Bildgebung mittels einer endoskopischen Nuklearsonde, etwa während eines operativen Eingriffs an einem Patienten. Die Nuklearsonde besitzt eine anisotrope Detektionscharakteristik, so dass nur Ereignisse aus einem bestimmten Raumwinkelbereich detektiert werden (siehe Fig. 2 und Fig. 8). Während der, typischerweise kontinuierlich durchgeführten, Messung werden die detektierten Werte der Sonde und/oder das daraus berechnete Bild regelmäßig von einer Auswerteeinheit bewertet und die Qualität des erzeugten Bildes durch Berechnung mindestens eines Qualitätsparameters ausgedrückt. Dieser Parameter kann dann zur Optimierung des Bilds benutzt werden, etwa um den Nutzer darauf hinzuweisen, dass er die Sonde noch an eine andere Lage/Position bewegen sollte zur Verbesserung der aufgenommenen Datenbasis. In einer aufwendigeren Variante kann die Qualitätsbeurteilung dazu benutzt werden, dem Benutzer gezielt eine bestimmte (oder mehrere verschiedene/alternative) neue Position/Lage der Sonde vorzuschlagen, um durch eine gezielte Positions- und oder Lageveränderung der Sonde durch den Nutzer die Qualität des aus den Sondendaten ermittelten 3D-Bildes zu erhöhen; ferner kann der Qualitätsparameter dazu benutzt werden, die Position der Sonde ohne Zutun eines Nutzers durch Aktuatoren zu verändern, etwa durch Ansteuerung eines Roboterarms, der die Nuklearsonde führt.

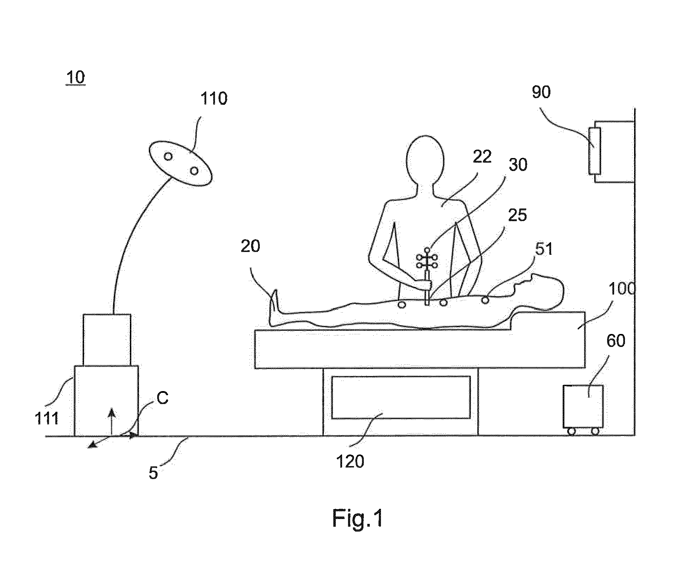

In Figur 1 ist ein System 10 zur endoskopischen nuklearen Bilderzeugung gemäß Ausführungsbeispielen gezeigt. Der Patient 20 und der Nutzer 22 sind nicht Bestandteile des Systems.

Die bewegliche endoskopische Nuklearsonde 25 umfasst einen Kollimator und einen Detektor zur Detektion von radioaktiver Strahlung. Die Sonde weist einen länglichen Körper mit einer Längsachse (X) auf, der in den Körper eines Patienten 20 eingeführt wird, etwa durch einen Trocar (nicht gezeigt). Dabei sind der Detektor und der Kollimator 26 am länglichen Körper der endoskopischen Nuklearsonde 25 angeordnet. Der Kollimator 26 hat ein Sichtfeld mit einer Achse (Y), wobei die Achse (Y) des Sichtfelds in Bezug auf die Achse (X) des länglichen Körpers der Sonde abgewinkelt ist. Dieses Sichtfeld ist schematisch in Fig. 2 gezeigt, der Bereich zwischen den gestrichelten Linien mit Mittelachse y. Dabei ist das Sichtfeld nicht nur in seitlicher Richtung des länglichen Körpers der Sonde ausgerichtet, sondern gleichzeitig anisotrop auf einen Winkelbereich mit Bezug auf die Längsachse X des länglichen Körpers beschränkt, in einem anschaulichen Bild ähnlich wie der Lichtkegel eines Leuchtturms.

Als Mittel zum Bestimmen der Position und Orientierung des länglichen Körpers der Sonde kommen unterschiedliche Techniken in Betracht. In Figur 1 ist ein optisches Tracking-System 110 gezeigt, das einen bzw. mehrere optische Marker 30 erfasst bzw. trackt und somit Informationen über Position und Lage der Sonde bereitstellt.

Die detektierten Informationen der Nuklearsonde 25 und des Trackingsystems 110 werden an eine Auswertungseinheit 60 übermittelt. Über dieses Schnittstellensystem werden Daten mit Informationen über die detektierte radioaktive Strahlung der Nuklearsonde übermittelt, sowie Informationen über die Ist-Position und Ist-Orientierung des länglichen Körpers der endoskopischen Nuklearsonde. Dies kann entweder kabelgebunden oder über Funkschnittstellen (nicht dargestellt) wie in Fig. 1 geschehen, es können auch Funk- und kabelgebundene Techniken kombiniert werden.

Die Auswertungseinheit 60 umfasst einen Datenspeicherabschnitt zum Speichern dieser Informationen. Mittels einer Synchronisiereinheit werden die Informationen über die Position und Orientierung des länglichen Körpers der endoskopischen Sonde mit der Information über die von der endoskopischen Nuklearsonde detektierte Strahlung synchronisiert, so dass die Auswertungseinheit 60 die Strahlungsinformation mit der Orts- und Ausrichtungsinformation verknüpfen kann, woraus sich mittels bekannter Algorithmen ein dreidimensionales Bild der Strahlungsquelle berechnen lässt (wie etwa bei der eingangs erwähnten Freihand SPECT-Bildrekonstruktion).

Während des obigen Prozesses werden eine Ist-Position und Ist-Orientierung des länglichen Körpers der endoskopischen Nuklearsonde 25 aus den Daten des Tracking-Systems 110 berechnet. Mittels der so gewonnenen Daten über die Position und Ausrichtung der Nuklearsonde 25 und die von ihr gelieferten Messdaten kann so ein dreidimensionales Bild der Verteilung des strahlenden Materials berechnet werden, also typischerweise eines im Gewebe des Patienten befindlichen (injizierten) radioaktiven Tracers, der sich in einem Tumor akkumuliert hat.

Anhand vorgegebener Algorithmen wird dann nach einer bestimmten Messzeit, beziehungsweise fortlaufend, ein Qualitätsparameter Q des so berechneten 3D-Bildes bzw. der Ortsverteilung des Tracermaterials berechnet. Dieser Parameter Q kann z.B. die Anzahl der detektierten Strahlungsereignisse (Counts) aus einem bestimmten Raumvolumen oder Raumwinkelelement und einer bestimmten Richtung berücksichtigen, unter Einbeziehung der Positions- und Lagedaten (=Pose) der Sonde. Sind etwa aus einem bestimmten Raumvolumen und einer bestimmten Richtung deutlich weniger Ereignisse erfasst als aus einem benachbarten Raumwinkelelement in der bestimmten Richtung, ist der für das erste Element berechnete Strahlungsverteilung mit einem höheren statistischen Fehler belastet, bzw ist die erzielbare Auflösung für das erste Element geringer.

Es gibt verschiedene Möglichkeiten für das Berechnen des Qualitätsparameters Q, bzw. kann Q je nach Verfahren z.B. ein Skalar, ein Vektor, ein Skalarfeld, ein Vektorfeld, ein Tensor oder eine 3D-Matrix sein. Q kann z.B. ausdrücken, wie groß die Varianz zwischen den Zählraten aus unterschiedlichen Raumwinkelelementen für jeweils unterschiedlichen Richtungen ist. Je größer also Q in diesem Fall ist, desto höher ist die Varianz der Qualität der erfassten Daten zwischen unterschiedlichen Raumwinkeln oder Teilvolumina aus bestimmten Richtungen, was im allgemeinen nicht wünschenswert ist, da dann bestimmte Teilvolumina z.B. eine zu geringe Bildauflösung bzw. ein zu hohes Bildrauschen aufweisen. Dies würde auch bedeuten, dass für mindestens eines der betrachteten Raumwinkelelemente die Aufnahmezeit deutlich geringer ist im Verhältnis zu anderen Bereichen, dort also die Qualität der Darstellung geringer ist. Ist also die Varianz Q in diesem Fall auf das Gesamtbild bezogen zu groß, entscheidet die Auswertungseinheit 60, dass Daten nacherhoben werden müssen. Der Bereich, für den dies zutrifft, ist dann aus der vorigen Berechnung bekannt. Somit kann die Auswertungseinheit berechnen, in welcher Richtung, bezogen auf die Ist-Position der Nuklearsonde, noch Daten erhoben werden sollten, um die Bildqualität zu verbessern bzw gleichzeitig Q zu senken.

In Ausführungsbeispielen können nun, abhängig von der Berechnung und Bewertung von Q (weitere Optionen für die Eigenschaften von Q siehe unten), mehrere Optionen realisiert sein. In einer einfachen Variante wird dem Nutzer der Sonde lediglich per binärem Signal (null/eins oder auch rot/grün) signalisiert, ob das bereits berechnete Bild den vorbestimmten Qualitätsanforderungen entspricht. Ist dies momentan nicht der Fall, kann eine rote Signallampe/LCD, die etwa an der Sonde oder an der Auswerteeinheit angebracht sein kann, dem Nutzer signalisieren, dass er die Sonde noch bewegen soll, ohne Information in welche Richtung bewegt oder wie gedreht werden soll. Stellt die Einheit dann fest, dass Q einen befriedigenden Wert erreicht hat, ändert sich die Anzeige auf grün. Selbstverständlich kann diese Signalisierung auf die unterschiedlichsten Weisen erfolgen, z.B. auch durch ein akustisches Signal oder durch eine Sprachausgabe in der Auswerteeinheit.

In einer weiteren Variante werden dem Nutzer Informationen über mögliche neue Posen der Sonde durch ein Ausgabegerät mitgeteilt, z.B. auf einem optischen Display, durch Sprachausgabe oder durch haptische Rückkopplung an der Sonde. In einer weiteren Variante wird die errechnete Korrekturinformation durch die Auswerteeinheit direkt zur Ansteuerung eines Aktuators benutzt, der die Pose der Sonde ändert. Dies kann z.B ein Roboterarm sein, der die Sonde führt, wobei der Arm gleichzeitig die Sensorik für das Tracken der Sonde umfassen kann.

Dabei eingesetzte Qualitätskriterien für den Parameter Q können beispielsweise sein (nicht-limitierend):
a) die skalare Dichte an Information für unterschiedliche Raumelemente (z.B. berechnet aus dem Einfluss des jeweiligen Raumelements in der gesammten bisherigen Datenaufnahme durch die Nuklearsonde) - in diesem Fall basiert Q auf einem 3D-Bild oder 3D-Skalarfeld
b) die vektorielle Dichte an Information für unterschiedliche Raumelemente (z.B. berechnet aus dem gerichteten Einfluss vom jeweiligen Raumelement in der gesamten bisherigen Datenaufnahme durch die Sonde) - in diesem Fall basiert Q auf einem 3D-Vektorfeld
c) die Separierbarkeit von rekonstruierten Strukturen in dem jeweiligen Raumelement, basierend auf den bisherigen aufgenommenen Daten - hierbei basiert Q auf einem 3D-Bild, dessen Detailschärfe und Auflösungsvermögen berechnet wird.
d) die Konditionsnummer der Rekonstruktionssystemmatrix, wie dem Fachmann bekannt - in diesem Fall ist Q ein Skalar.
e) das Spektrum der Eigenwerte oder Singularwerte der Rekonstruktionssystemmatrix - hier basiert Q auf einem Vektor.

Weitere Verfahren zur Bestimmung von Qualitätsparametern Q eines computergenerierten Bilds aus einer Nuklearsonde sind in der deutschen Patentanmeldung 102008025151.8 etwa auf den Seiten 37 bis 42 beschrieben, deren diesbezügliche Lehre hiermit durch Verweis komplett einbezogen ist.

Abhängig davon, ob sich aus dem errechneten Q nach einem der obigen oder ähnlichen bzw. davon abgeleiteten Verfahren eine mögliche Verbesserung zumindest eines Teilbereichs des Bilds ergibt, kann nun eine der oben skizzierten Handlungsanweisungen gewählt werden - also Signalisierung an den Nutzer ob weitere Bewegung oder nicht; Ausgabe von ein oder mehreren neuen Sollposen bzw. der Vektoren, die zu diesen Posen führen; oder die Ansteuerung eines oder mehrere Aktuatoren zur Bewegung der Sonde.

Im Fall, der Option der Ausgabe der Soll-Pose bzw. der entsprechenden Bewegungsinformationen wird aus den aus Q abgeleiteten Informationen nun eine Soll-Position und Soll-Orientierung des länglichen Körpers der endoskopischen Nuklearsonde 25 berechnet; wie oben beschrieben mit der Nebenbedingung, dass eine entsprechende Positionsänderung der Nuklearsonde zu einer Verbesserung des Qualitätswerts Q führt; was gleichzeitig eine Steigerung der Bildqualität bedeuten soll bzw. bedeutet. Mittels eines Ausgabesystems, zB. Dem optischen Display 90, werden die berechneten Soll-Informationen, nach Umwandlung in Anweisungen zum Bewegen der endoskopischen Nuklearsonde, an einen Benutzer ausgegeben. Eine mögliche entsprechende Darstellung ist in Fig. 3 gezeigt.

Zur Bestimmung der genannten Soll-Werte für die Pose der Nuklearsonde 25 können mehrere Informationen benutzt werden, wie etwa das Bewegungsvolumen, in dem die Nuklearsonde bewegt werden kann, die möglichen Richtungen, in die die Nuklearsonde gerichtet werden kann (u.a. abhängig von der durch die präoperativen Bilddaten bekannte Anatomie des Patienten), die Lage bzw. Pose des Patienten und der Organe im Körper, die erwartete Radioaktivitätsverteilung im Körper, etc. Diese Informationen können aus präoperativen Daten des Patienten (registriert oder nicht), statistischen Modellen, Informationen der Bewegung der Nuklearsonde oder weiterer endoskopischer Instrumente, etc., gewonnen werden. Diese Informationen können zum Beispiel benutzt werden, um die Plausibilität der Soll-Werte zu überprüfen, oder sie zu wichten. So muss etwa sichergestellt sein, dass nicht eine Pose vorgeschlagen wird, bei der sich der Ort der Sonde mit einem Organ überschneiden würde.

Das Ausgabesystem 90 ist typischerweise ein Bildschirm, kann aber auch eine Vielzahl anderer Varianten umfassen, z.B. ein Sprachausgabesystem oder eine haptische Ausgabe mittels Kraft-Rückkopplung über Aktuatoren in der Nuklearsonde selbst, etwa im Griff. Auf diese Weise kann der Nutzer gemäß den übermittelten Informationen die Sonde an die Soll-Position und Soll-Orientierung bewegen, so dass zusätzliche Ereignisse die Zählrate für den betreffenden Raumwinkel verbessern können.

Ein Beispiel für die Darstellung einer Korrekturinformation an einen Nutzer 22, die typischerweise auf dem Display 90 dargestellt wird, ist in Fig. 3 gezeigt. Der Kreis mit dem Kreuz 50 symbolisiert dabei die Sonde und die Markierung 55 die Orientierung dieser gegenüber Ihrer Längsachse. Der runde Pfeil 52 zeigt dem Nutzer an, wie die Sonde um ihre Längsachse gedreht werden soll, und der lineare Pfeil 54 zeigt eine Verschiebung an, also dass der Nutzer den Griff in diese Richtung bewegen soll. Die Anzeige kann in Echtzeit auf Aktionen eines Benutzers reagieren, so dass bei korrekter Bewegung die entsprechenden Pfeile kleiner werden und bei Erreichen der neuen Sollposition verschwinden. Bei falscher oder nicht ganz korrekter Bewegung durch den Nutzer kann es dagegen sein, dass ein Pfeil größer wird oder die Richtung ändert. In weiter unten beschriebenen Ausführungsbeispielen kann die Korrekturinformation direkt in die Steuerung eines die Sonde haltenden Roboterarms fließen, so dass der menschliche Faktor als Fehlerquelle ausgeschaltet wird.

Wie in Fig. 1 gezeigt, besitzt das optische Trackingsystem ein Koordinatensystem C. Die Lage des Operationstisches 100 mit Bezug auf das Trackingsystem ist typischerweise bekannt bzw. kann zuvor ausgemessen werden. Wenn der Patient 20 in einer definierten Art und Weise auf dem OPTisch liegt und unbeweglich, etwa weil narkotisiert oder fixiert ist, und der OPTisch fix gegenüber dem Trackingsystem ist, kann daher von einer stationären Lage des Patenten 20 ausgegangen werden. Es ist notwendig, die exakten Außenabmessungen des Patienten bzw. dessen Lage auf dem Tisch 100 und seine exakte Position zu kennen, um eine Bildgebung mittels der Detektordaten der Sonde in einer Kombination mit, zum Beispiel, präoperativen Bildern, wie etwa CT-Bildern zu errechnen. Wird die Lage des Patienten nicht mit in das Verfahren einbezogen, wird das berechnete 3D-Bild, zum Beispiel eines Tumors, ohne Bezug auf den Körper des Patienten auf einem Bildschirm 90 dargestellt. Das Trackingsystem 110 kann in Ausführungsbeispielen auch in beliebigen anderen Trackingtechniken ausgeführt sein, zum Beispiel als elektromagnetisches Trackingsystem, als mechanisches Trackingsystem (wie etwa bei Robotersystemen oder bei Positionierungsarmen), als Beschleunigungsensor- oder Gyroskop-basiertes Trackingsystem, als Schall-Trackingsystem, als Ultraschall-Trackingsystem, oder als ein radioaktives Trackingsystem bzw. RF-basiertes Trackingsystem.

Eine Möglichkeit, präoperative Daten, zum Beispiel Computertomographie- (CT-) oder Kernspin- (MRI-)Bilder in diesem Verfahren zu nutzen, wäre, diese bereits vor der Operation mit einem Marker versehen aufzunehmen, und während der oben beschriebenen Anwendung des Verfahrens mit mindestens einem Marker 51 (siehe Figur 1) am Körper des Patienten rechnerisch in der Auswertungseinheit 60 zur Deckung zu bringen.

Eine weitere Möglichkeit, präoperative Daten in diesem Verfahren zu nutzen, wäre die intraoperative Aufnahme von der Oberfläche vom Patient durch ein optisches oder haptisches Abtastsystem und die Registrierung dieser Oberfläche mit der aus den präoperativen Daten berechneten Oberfläche des Patienten. Dazu kann z.B. auch das optische Trackingsystem 110 aus Fig. 1 gleichzeitig ausgelegt sein.

In Ausführungsbeispielen ist optional mindestens eine Referenz, in Form des mindestens einen Markers 51, an dem bzw. in Bezug auf den Patienten 20 fixiert. Die Position dieser Referenz wird während der Anwendung des erfindungsgemäßen Verfahrens über Mittel zum Bestimmen der Position und Orientierung der Referenz bestimmt. Diese Mittel können etwa ein optisches Trackingsystem sein, so kann etwa zweckmäßigerweise das Trackingsystem 110 auch zur Ortsbestimmung des/der Marker 51 als Referenz(en) genutzt werden. Die Informationen über die Lage der Referenz(en) (und gegebenenfalls deren Orientierung, z.B. bei räumlich ausgedehnten, nicht punktartigen Markern) wird über Schnittstellen (in Figur 1 Funkschnittstellen) an die Auswerteeinheit 60 gesendet.

Diese Positions- und Lagedaten der Referenz können von und in der Auswerteeinheit 60 mit den anderen im System verarbeiteten Daten, nämlich Ort und Lage der Nuklearsonde sowie die Zählinformationen der Sonde, synchronisiert werden. Auf diese Weise kann selbst einer Lageänderung des Patienten 20 während einer Operation Rechnung getragen werden. Die Aufnahme der verschiedenen Informationen, also Zählinformationen der Nuklearsonde, deren Lage- und Ortsinformationen mittels des Trackingsystems 110 sowie gegebenenfalls die Lageinformationen des Patienten 20 über die Referenzen 51, werden dabei typischerweise fortlaufend erfasst und miteinander synchronisiert. Die aus den Zählraten der Sonde und den Lage- bzw Positionsdaten errechneten 3D-Bildinformationen können dabei mit beliebigen Bildern des Körpers des Patienten überlagert und auf einem Bildschirm 90 (oder etwa einer 3D-Brille) ausgegeben werden. Solche präoperativen Daten können z.B. CT-Bilder oder MRI-Bilder sein. Um diese mit den Bilddaten der Sonde überein zu bringen, also die Daten so zu synchronisieren, dass z.B. ein radioaktiv markierter, von der Nuklearsonde detektierter Tumor an der richtigen Stelle in das CT-Bild eingeblendet wird, bestimmt die Auswerteeinheit 60 ständig bzw. in regelmäßigen Intervallen die Ist-Position und Ist-Orientierung des länglichen Körpers der endoskopischen Nuklearsonde 25 relativ zu der Referenz, bzw. den Markern 51.

Das Ausgabesystem 90 der Auswerteeinheit 60 kann somit zum Ausgeben einer dreidimensionalen Wiedergabe der Informationen über die Ist-Position und Ist-Orientierung des länglichen Körpers der endoskopischen Nuklearsonde 25, und der Informationen über die Ist-Position und Ist-Orientierung der Referenz 51 und/oder Information über die detektierte Strahlung ausgelegt sein.

Das Ausgabesystem 90 ist typischerweise ein Display, das ein visuelles Display, ein haptisches Display, ein akustisches Display oder eine Kombination mehrerer dieser Displays sein kann. Insbesondere kann das Display dazu eingerichtet sein, wie oben beschrieben Anweisungen an einen Benutzer wiederzugeben. Diese umfassen typischerweise den Verweis auf eine weitere mögliche Soll-Position und Soll-Orientierung des länglichen Körpers der endoskopischen Nuklearsonde, um die gegenwärtige Datenlage zu verbessern, bzw. entsprechende Korrekturanweisungen zur Bewegung der Sonde 25 wie in Figur 3 dargestellt zu liefern.

Die endoskopische Nuklearsonde 25 ist dabei typischerweise zur Detektion der radioaktiven Strahlung eines radioaktiven Markers im Inneren eines Körpers ausgelegt, vorzugsweise der Detektion von Gamma-Strahlung, Beta-Strahlung, oder beidem.

Bei der Berechnung möglicher Soll-Positionen und Soll-Orientierungen der Nuklearsonde kann in Ausführungsbeispielen die Position eines Trocars, durch das die Sonde in den Körper des Patienten eingeführt ist, bzw. generell mögliche Bewegungen der Nuklearsonde im Körper berücksichtigt werden. Wenn etwa die Sonde in einem Hohlraum innerhalb des Körpers eingeführt ist, so dass sich der den Detektor/Kollimator umfassende Teil der Sonde in dem Hohlraum bewegt, kann die Information über den Hohlraum bei der Berechnung möglicher Alternativpositionen berücksichtigt werden. Diese Information kann etwa durch die Auswerteeinheit aus den präoperativen Bilddaten extrahiert werden. Zudem können typische mögliche Bewegungsparameter der Sonde bezüglich des Trocars in der Auswerteeinheit gespeichert sein. Eine ähnliche Strategie kann sogar, ohne Erfassung der Position des Trocars, aus den bisherigen Bewegungen der Nuklearsonde abgeleitet werden.

Das Detektionsfeld der Nuklearsonde 25 kann in Bezug auf die Längsachse (X) in einem beliebigen Ausschnitt des Winkelbereichs von 0 bis 180 Grad angeordnet sein, als nicht-limitierende Beispiele z.B. 0 Grad bis 90 Grad, 0 Grad bis 120 Grad, oder 10/20 Grad bis 180 Grad. Die Nuklearsonde kann in Ausführungsbeispielen in einem Endoskop eingesetzt werden, wobei das Endoskop (nicht-limitierend) ein Laparoskop, ein Laryngoskop, ein Rynoskop, ein Arthroskop, ein Epiduroskop, ein Ösophagoskop, ein Gastroskop, ein Enteroskop, ein Anoskop, ein Rektoskop, ein Colonoskop, ein Symodoiskop, ein Proktoskop, ein Zystoskop, ein Gynoskop, ein Kolposkop, ein Histeroskop, ein Falloposkop, ein Bronchoskop, ein Thorakoskop, ein Mediastinoskop, ein Otoskop, oder ein Duktoskop sein kann. Diese nichtabschließende Aufzählung ist grundsätzlich für alle in dieser Schrift genannten Endoskope gültig.

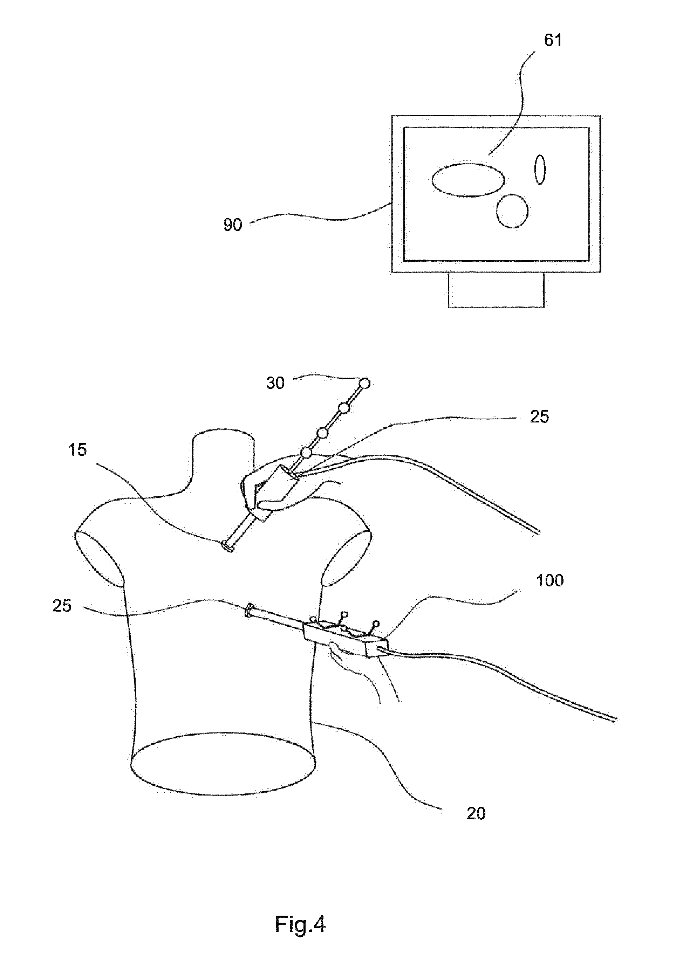

Zusätzlich zu der Nuklearsonde kann mindestens ein weiteres, positioniertes Weißlicht- und/oder ein Fluoreszens-Endoskop 100 eingesetzt werden, das optische Informationen an das Auswertesystem überträgt, das heißt an die Auswerteeinheit 60, siehe Figur 4. Deren Bildinformationen können über ein visuelles Display 90 ausgegeben werden, um Virtual- bzw. Augmented-Reality-Darstellungen von dreidimensionalen Bildern und Anweisungen an den Benutzer aus der Perspektive dieses Weißlicht- und/oder Fluoreszens-Endoskops 100 zu erzeugen. Das heißt, die Bildinformationen des/der zusätzlichen Endoskope 100 werden als eine mögliche Bildquelle genützt, die mit den Bildinformationen aus der Nuklearsonde 25 und optional mit weiteren präoperativen Bildern 61 kombiniert dargestellt werden.

Die auf einem Display 90 dargestellte Virtual Reality eines der positionierten endoskopischen Instrumente 100, entweder des zusätzlichen optischen, oder der Nuklearsonde 25, kann in Ausführungsbeispielen in Bezug zu den dreidimensionalen Bildern aus der Perspektive der jeweils anderen dargestellt werden. Dabei können die mit der Nuklearsonde gewonnenen Bildinformationen auch mit Ultraschallbildern kombiniert visuell dargestellt werden, wobei das zusätzliche endoskopische Instrument 100 folglich eine endoskopische Ultraschallsonde ist. Voraussetzung für die erwähnten Bildfusionierungen und die Erfassung von zusätzlichen Positions- und Orientierungsinformation sowie Patienten und Organlagen, ist eine Kallibrierung der Position, Orientierung und äquivalenten Optik der Nuklearsonde mit den jeweiligen Zusatz-Bilderfassungssystemen. Dies kann per Konstruktion erfolgen, durch entkoppeltes Nachführen der Zusatzbilderfassungssysteme, durch Kalibrierungsmittel auf Basis bekannter Strukturen (wie etwa Kalibrierungsphantomen) oder einer beliebigen Kombination der vorgenannten erfolgen.

In Ausführungsbeispielen kann das erfindungsgemäße bildgebende System einen weiteren externen Detektor 120 (siehe Figur 1) außerhalb des Patienten 20 beinhalten. Dieser ist vorzugsweise eine Gamma-Kamera, oder eine PET- oder Compton-Kamera, die mit dem Detektor der endoskopischen Nuklearsonde 25 in Koinzidenz arbeitet. Somit kann zusätzliche Information für die Erzeugung der dreidimensionalen Wiedergabe bereitstellt werden, wobei der externe Detektor vorzugsweise unter (siehe Figur 1), über oder an der Seite des Patienten 20 oder um den Patienten 20 herum angeordnet ist.

Wenn das System 10, wie oben bereits beschrieben, auch zur Wiedergabe präoperativ gewonnener Bilddaten eingerichtet ist, können diese Daten vorzugsweise aus der Perspektive eines der genannten endoskopischen Instrumente 100 dargestellt werden - beziehungsweise können diese Daten als Vorlage für die Erzeugung der dreidimensionalen Wiedergabe verwendet werden.

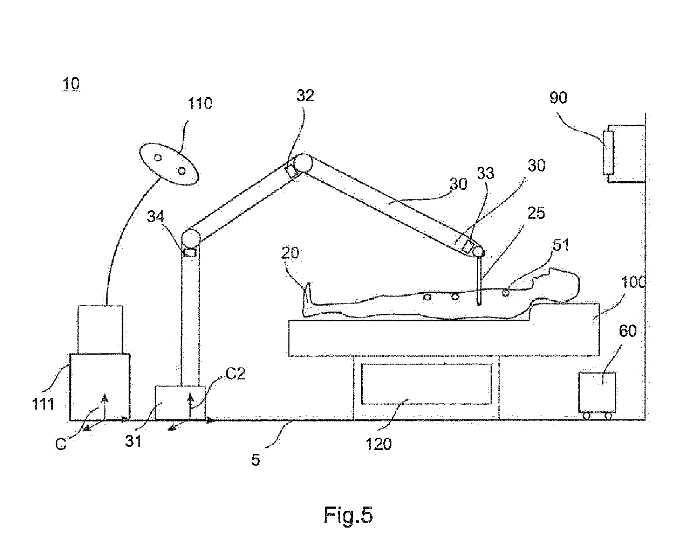

In Ausführungsbeispielen, wie in Fig. 5 gezeigt, kann die Nuklearsonde auch von einem Roboterarm 30 gehalten bzw. geführt werden. Der Roboterarm ist dabei typischerweise, etwa per Funkschnittstelle, mit der Auswerteeinheit 60 verbunden, so dass die möglichen neuen Soll-Positionsdaten und Soll-Lagedaten direkt von der Auswerteeinheit 60 als Steuerdaten an den Roboterarm übertragen werden, der die Bewegungen ausführt, um so die Bildgebung durch die Nuklearsonde 25 zu verbessern. Weiterhin bzw. alternativ dazu kann auch eines oder mehrere der endoskopischen Instrumente 100 von einem zweiten Roboterarm gehalten werden. Der Arm besitzt Sensoren 32, 33, 34 zur Lageerkennung der Sonde 25. Sein Koordinatensystem C2 (es ist dem Fachmann klar, dass Koordinatensysteme hier beliebig gesetzt werden können, solange ihre Beziehung zueinander bekannt ist) dient als Referenz für die Lage der Sonde 25. Das System 110 kann hier etwa zur Aufnahme der Lage und Oberfläche des Patienten 20 dienen.

In Ausführungsbeispielen kann die endoskopische Nuklearsonde in ein Endoskop eingesetzt werden. Typische Endoskope für diesen Zweck sind (nicht-limitierend) ein Laparoskop, ein Laryngoskop, ein Rynoskop, ein Arthroskop, ein Epiduroskop, ein Ösophagoskop, ein Gastroskop, ein Enteroskop, ein Anoskop, ein Rektoskop, ein Colonoskop, ein Symodoiskop, ein Proktoskop, ein Zystoskop, ein Gynoskop, ein Kolposkop, ein Histeroskop, ein Falloposkop, ein Bronchoskop, ein Thorakoskop, ein Mediastinoskop, ein Otoskop, oder ein Duktoskop.

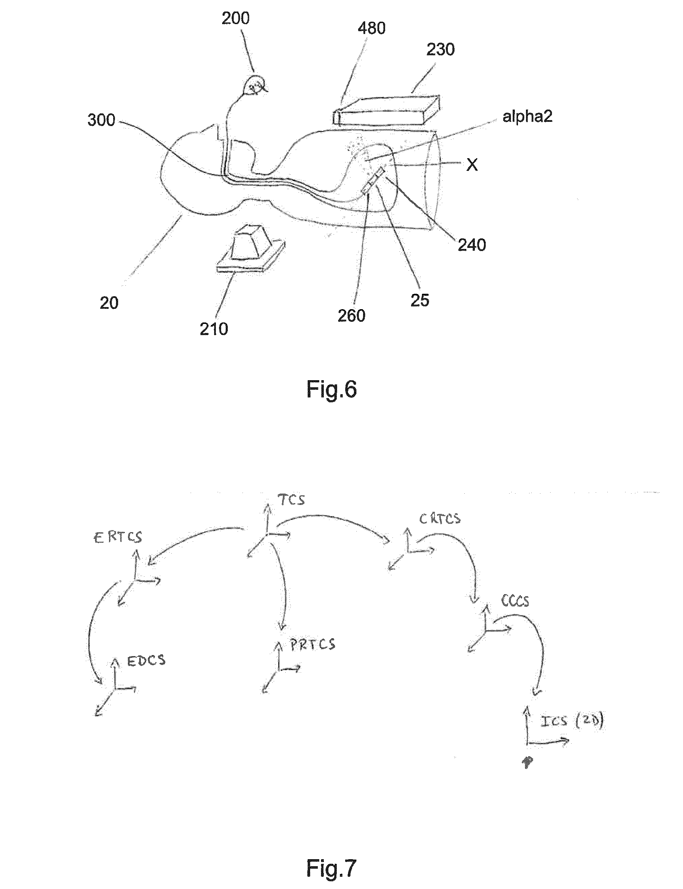

Fig. 6 zeigt ein Ausführungsbeispiel, bei dem die Nuklearsonde 25 komplett in dem Patienten 20 befindlich ist und über eine mechanische oder elektrische Steuerung 200 durch ein Gastroskop 300 von außen bewegt/ausgerichtet wird. Ein z.B. elektromagnetisches Trackingsystem 210 überwacht die Lage der Nuklearsonde, die eine Achse X und ein dreidimensionales Blickfeld alpha2 hat. Eine Gammakamera 230 kann in Koinzidenz mit der Nuklearsonde 25 arbeiten. Die Sonde 25 ist für das Tracking mit einem elektromagnetischen Sensor 240 versehen. Sie hat außerdem eine zusätzlichen Ultraschallgeber und -sonde 260, um zusätzliche Bildinfromation zu generieren. Die Nuklearsonde ist ein länglicher Körper am Ende des Endoskops (Gastroskop) 300, das durch die Speiseröhre eingeführt ist. In diesem endoskopischen System (mit einem Gastroskop), das elektromagnetisch getrackt wird, ist also neben der Nuklearsonde im Endoskop auch eine Gamma-Kamera 230 über dem Patient, die weitere Information für die Bildgebung erbringt. Getrackt durch System 210 werden dabei die Nuklearsonde (der längliche Körper am Ende des Gastroskops 300) und die Gamma-Kamera 230.

In Fig. 7 ist die Berechnung der relativen Pose (= Position und Orientierung) der Nuklearsonde gegenüber der Referenz 51 (am Patient, siehe etwa Fig. 1). Dies geschieht über Transformationsmatrizen in homogenen Koordinaten, bei denen ein 3D-Punkt als ein 4D-Vektor dargestellt wird. Jede von diesen 4x4-Matrizen wird als "T" gekennzeichnet. Die Richtung der Transformation ist vom Koordinatensystem unten rechts in Richtung Koordinatensystem oben links. Die unterschiedlichen Koordinatensystemen sind auch darstellt als 3 Pfeilen, da sie hier alle 3D-Koordinatensystemen sind. Die Koordinatensysteme sind wie folgt bezeichnet:

TCS = Tracking (System) Coordinate System

RCS = (Patient) Reference Coordinate System

PCS = (Nuclear) Probe Coordinate System

DCS = Detector Coordinate System

Die Transformation PCS_T_TCS und die Transformation RCS_T_TCS werden vom Trackingsystem 110, 210 (vgl. Fig.1, Fig. 6) in Echtzeit geliefert. Die Transformation DCS_T_PCS wird durch eine einmalige Kalibrierung bestimmt. z.B. eine "Hand-Eye-Kalibrierung".

In Figur 8 ist eine typische laparoskopische Nuklearsonde 25 gemäß Ausführungsbeispielen gezeigt, wie sie etwa in Fig. 1 und Fig. 9 gezeigt ist. Sie umfasst ein Gehäuse 400, an dessen Ende ein Wolfram-Kollimator 26, 426 angebracht ist. Die Sonde hat eine Längsachse X. Der Kollimator hat eine seitwärts gerichtete Öffnung 420, die den Detektionswinkel alpha begrenzt, bzw. die Detektionscharakteristik durch ein Blickfeld festlegt. In dem Kollimator 426, 26 befindet sich ein Szintillator-Kristall 440. Die gezeigte laparoskopische Gammasonde weist ein Fenster mit Öffnungswinkel von ca. 70 Grad auf. Der Silizium-Photomultiplier 480 neben dem Szintillator-Kristall 440 wandelt das vom Szintillator erzeugte, einfallende Licht in Elektrizität um. Durch ein Kabel 450 ist der Silizium-Photomultiplier mit der Außenwelt verbunden. Das Blickfeld mit dem Öffnungswinkel bzw. Winkelbereich alpha hat eine Mittelachse Y, die geneigt zur Längsachse X der Sonde hat.

Figur 9 zeigt die Koordinaten, die benötigt werden, um das 3D-Nuklearbild eines Systems (wie in Fig. 1) in die Perspektive eines weiteren Videoendoskops (das ein 2D-Bild liefert, wie etwa in Fig. 4 möglich) darzustellen. Das Trackingsystem (mit Koordinatensystem TCS) liefert die Pose des Nuklearsonden-Referenztargets 30 (Koordinatensystem ERTCS), des Patienten-Referenztargets 51 (Koordinatensystem PRTCS) und vom Videoendoskop-Referenztarget (CRTCS, nicht dargestellt, in Fig. 4 innerhalb des Körpers). Durch Kalibrierung der Nuklearsonde 25 bekommt man die Pose des Detektors (Koordinatensystem EDCS). Durch Kalibrierung ergibt sich dann auch die Pose des Kamerazentrums des Videoendoskops (Koordinatensystem CCCS) und die Pose der Bildebene (ICS). Dabei ist zu berücksichtigen, dass das Koordinatensystem des Videobildes nur in 2 Dimensionen ist. Somit ist die Transformation von CCCS auf ICS eine Projektion von 3D auf 2D, also eine 3x4 Matrix.

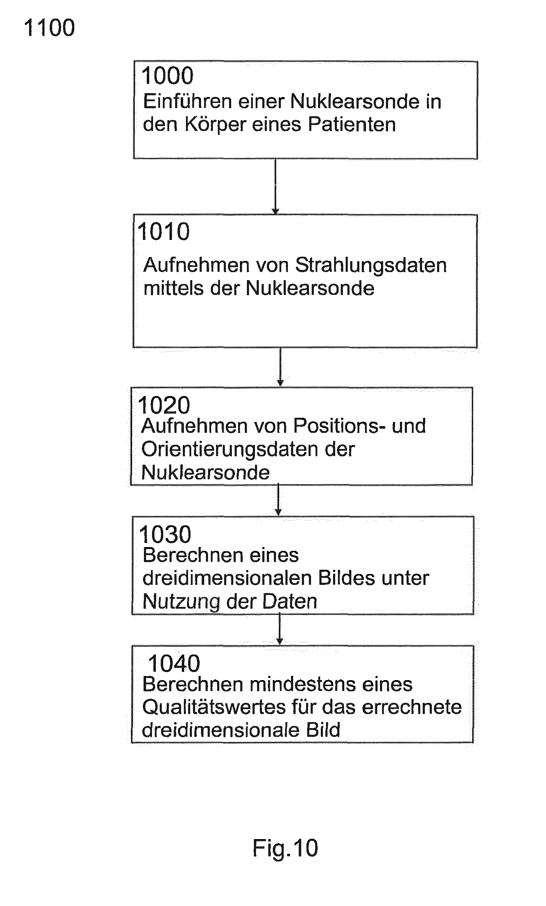

Figur 10 zeigt ein Verfahren 1100 gemäß Ausführungsbeispielen. Es umfasst Einführen einer Nuklearsonde in den Körper eines Patienten in Block 1000; Aufnehmen von Strahlungsdaten mittels der Nuklearsonde in Block 1010; Aufnehmen von Positions- und Orientierungsdaten der Nuklearsonde in Block 1020; Berechnen eines dreidimensionalen Bildes unter Nutzung der Daten in Block 1030; und Berechnen mindestens eines Qualitätswertes für das errechnete dreidimensionale Bild in Block 1040. Darauf kann gemäß Ausführungsbeispielen folgen: das Anzeigen einer binären Information - ob die Sonde weiter bewegt werden soll oder nicht - an einen Nutzer, der die Sonde führt; oder das Anzeigen von Vorschlägen für mögliche Bewegungen der Nuklearsonde; und/oder die Anzeige von Sollkoordinaten und einer Soll-Lage der Sonde (kombiniert: Soll-Pose); und/oder das Ansteuern eines Aktuators, bevorzugt eines Roboterarms, um die Pose der Sonde zu ändern.

Während das Vorangehende auf Ausführungsformen der Erfindung gerichtet ist, können andere und weitere Ausführungsformen der Erfindung durch Kombinationen der beschriebenen aufgestellt werden, ohne vom Schutzbereich der Erfindung abzuweichen, der durch die nachfolgenden Ansprüche bestimmt wird.

## Patentansprüche

1. System zur endoskopischen nuklearen Bilderzeugung, welches umfasst:
- eine bewegliche endoskopische Nuklearsonde, welche einen Kollimator und einen Detektor zur Detektion von radioaktiver Strahlung umfasst, wobei die endoskopische Nuklearsonde einen länglichen Körper mit einer Längsachse (X) aufweist, der ausgelegt ist in den Körper eines Patienten eingeführt zu werden, wobei der Detektor und der Kollimator am länglichen Körper der endoskopischen Nuklearsonde angeordnet ist, und wobei der Kollimator ein Sichtfeld mit einer Achse (Y) aufweist, wobei die Achse (Y) des Sichtfelds des Kollimators in Bezug auf die Achse (X) des länglichen Körpers der endoskopischen Nuklearsonde abgewinkelt ist;
- Mittel zum Bestimmen der Position und Orientierung des länglichen Körpers der endoskopischen Nuklearsonde;
- eine Auswertungseinheit, welche umfasst:
i) ein Schnittstellensystem zur Übermittlung von Daten mit Information über die detektierte radioaktive Strahlung, von Information über die Position und Orientierung des länglichen Körpers der endoskopischen Nuklearsonde an die Auswertungseinheit,
ii) einen Datenspeicherabschnitt zum Speichern der Informationen;
iii) Mittel zum Synchronisieren der Information über die Position und Orientierung des länglichen Körpers der endoskopischen Nuklearsonde mit der Information über die von der endoskopischen Nuklearsonde detektierte Strahlung;
iv) Mittel zur rechnerischen Bestimmung einer Ist-Position und Ist-Orientierung des länglichen Körpers der endoskopischen Nuklearsonde;
v) einen Programmspeicherabschnitt mit einem Programm zum wiederholten Ermitteln mindestens eines Qualitätswerts Q hinsichtlich der Bilderzeugung aus den genannten Informationen;
vi) ein Ausgabesystem zum Ausgeben einer dreidimensionalen Wiedergabe der Informationen über die Ist-Position und Ist-Orientierung des länglichen Körpers der endoskopischen Nuklearsonde und der Information über die detektierte Strahlung.

2. System nach Anspruch 1, wobei die Auswerteeinheit weiter umfasst:
Mittel zum Berechnen einer Soll-Position und Soll-Orientierung des länglichen Körpers der endoskopischen Nuklearsonde, wobei diese Daten unter der Nebenbedingung bestimmt werden, dass eine entsprechende Platzierung des länglichen Körpers der endoskopischen Nuklearsonde zu einer Verbesserung des mindestens einen Qualitätswerts Q führt; sowie ein Ausgabesystem zum Ausgeben einer Anweisung zum Bewegen der endoskopischen Nuklearsonde an die Soll-Position und Soll-Orientierung an einen Benutzer oder Roboter.

3. System nach Anspruch 1 oder 2, weiter umfassend:
- eine Referenz, die in Bezug auf den Patienten fixiert ist;
- Mittel zum Bestimmen der Position und Orientierung der Referenz,
wobei das Schnittstellensystem der Auswerteeinheit weiter ausgelegt ist zur Übermittlung von Daten mit Informationen über die Position und Orientierung der Referenz an die Auswertungseinheit,
und wobei die Mittel zum Synchronisieren der Auswerteeinheit weiter zum Synchronisieren der Information über die Position und Orientierung der Referenz ausgelegt sind;
und die Auswerteeinheit Mittel zum Bestimmen einer Ist-Position und Ist-Orientierung des länglichen Körpers der endoskopischen Nuklearsonde relativ zu der Referenz aufweisen;
und das Ausgabesystem weiter zum Ausgeben einer dreidimensionalen Wiedergabe der Informationen über die Ist-Position und Ist-Orientierung des länglichen Körpers der endoskopischen Nuklearsonde und der Informationen über die Ist-Position und Ist-Orientierung der Referenz und/oder Information über die detektierte Strahlung ausgelegt ist,
bevorzugt weiter umfassend ein Display zum Darstellen der erzeugten dreidimensionalen Wiedergabe, wobei das Display bevorzugt ausgewählt ist aus der Gruppe bestehend aus einem visuellen Display, einem haptischen Display, einem akustischem Display und einer Kombination mehrerer dieser Displays, wobei das Display bevorzugt ferner eingerichtet ist, Anweisungen an einen Benutzer wiederzugeben, wobei die Anweisungen bevorzugt den Verweis auf eine weitere mögliche Soll-Position und Soll-Orientierung des länglichen Körpers der endoskopischen Nuklearsonde umfassen.

4. System nach einem der Ansprüche 1 bis 3, wobei die endoskopische Nuklearsonde zur Detektion der radioaktiven Strahlung eines radioaktiven Markers im Inneren eines Körpers ausgelegt ist, vorzugsweise der Detektion von Gamma-Strahlung, Beta-Strahlung, oder beiden.

5. System nach einem der Ansprüche 1 bis 4, wobei das Mittel zum Berechnen zusätzlich möglicher Soll-Positionen und Soll-Orientierungen des länglichen Körpers der endoskopischen Nuklearsonde, vorzugsweise in Bezug zu der Referenz, bei der Berechnung Informationen betreffend die Position eines Trocars und/oder mögliche Bewegungen der endoskopischen Nuklearsonde berücksichtigt.

6. System nach einem der vorhergehenden Ansprüche, wobei die Referenz an dem Patienten fixiert ist oder an einem anderen Element fixiert ist, welches relativ zu dem Patienten fixiert ist.

7. System nach einem der vorhergehenden Ansprüche, wobei das Detektionsfeld der endoskopischen Nuklearsonde in Bezug auf die Längsachse (X) in einem beliebigen Ausschnitt des Winkelbereichs von 0 bis 180 Grad angeordnet ist.

8. System nach einem der vorhergehenden Ansprüche, wobei die endoskopische Nuklearsonde in einem Endoskop einsetzbar ist, wobei das Endoskop ein Laparoskop, ein Laryngoskop, ein Rynoskop, ein Arthroskop, ein Epiduroskop, ein Ösophagoskop, ein Gastroskop, ein Enteroskop, ein Anoskop, ein Rektoskop, ein Colonoskop, ein Symodoiskop, ein Proktoskop, ein Zystoskop, ein Gynoskop, ein Kolposkop, ein Histeroskop, ein Falloposkop, ein Bronchoskop, ein Thorakoskop, ein Mediastinoskop, ein Otoskop, oder ein Duktoskop ist.

9. System nach einem der vorhergehenden Ansprüche, wobei das System des Weiteren zumindest eines der Folgenden umfasst:
(a) ein positioniertes Weißlicht- und/oder ein Fluoreszens-Endoskop,
(b) ein visuelles Display für Virtual- oder Augmented-Reality-Darstellungen von dreidimensionalen Bildern und Anweisungen an den Benutzer aus der Perspektive dieses Weißlicht- und/oder Fluoreszens-Endoskops;
(c) ein weiteres positioniertes endoskopisches Instrument, und ein visuelles Display zum Darstellen von Virtual-Reality und Anweisungen aus dessen Perspektive, wobei die Virtual Reality optional eines der positionierten endoskopischen Instrumente in Bezug zu den dreidimensionalen Bildern aus der Perspektive der jeweils anderen dargestellt werden kann, und wobei optional eines der endoskopischen Instrumente eine Ultraschallsonde beinhaltet, so dass berechnete Bildinformationen der Ultraschallsonde gemeinsam mit den Bildinformationen der Nuklearsonde auf einem Display dargestellt werden können;
(d) einen externen Detektor, vorzugsweise eine Gamma-Kamera, eine PET- oder Compton-Kamera, die mit dem Detektor in Koinzidenz arbeitet, welcher zusätzliche Information für die Erzeugung der dreidimensionalen Wiedergabe bereitstellt, wobei der externe Detektor vorzugsweise unter, über oder an der Seite des Patienten oder um den Patienten herum angeordnet ist;
(e) einen Roboter, der zum Halten der endoskopischen Nuklearsonde und / oder eines oder mehrerer der endoskopischen Instrumente konfiguriert ist.

10. System nach einem der vorhergehenden Ansprüche, wobei das System des Weiteren zur Wiedergabe präoperativ gewonnener Daten eingerichtet ist, und wobei diese Daten vorzugsweise aus der Perspektive eines der genannten endoskopischen Instrumente dargestellt werden, oder sie als Vorlage für die Erzeugung der dreidimensionalen Wiedergabe oder zur Berechnung der Soll-Position und Soll-Orientierung des länglichen Körpers der endoskopischen Nuklearsonde verwendet werden kann.

11. System nach einem der vorhergehenden Ansprüche, wobei das Mittel zum Bestimmen der Ist-Position und Ist-Orientierung des länglichen Körpers der endoskopischen Nuklearsonde ein elektromagnetisches System, ein optisches Trackingsystem, ein mechanisches Trackingsystem, ein Beschleunigungsensor- oder Gyroskop-basiertes Trackingsystem, ein Schall-Trackingsystem, ein Ultraschall-Trackingsystem, ein radioaktives Trackingsystem oder ein RF-basiertes System ist.

12. Endoskopische Nuklearsonde, umfassend:
- einen länglichen Grundkörper,
- einen Kollimator,
- einen Detektor zur Detektion von radioaktiver Strahlung, wobei die endoskopische Nuklearsonde eingerichtet ist, mit dem länglichen Grundkörper in den Körper eines Patienten eingeführt zu werden, und wobei der Kollimator am distalen Ende angeordnet ist, und wobei der Kollimator ein Sichtfeld mit einer Achse (Y) aufweist, wobei die Achse (Y) des Sichtfelds des Kollimators in Bezug auf die Achse (X) des länglichen Körpers der endoskopischen Nuklearsonde abgewinkelt ist, so dass der Detektionsbereich der Sonde für radioaktive Strahlung in einem Winkelbereich von 0 Grad bis 180 Grad zur Längsachse liegt, und wobei die Detektionscharakteristik anisotrop in Bezug auf eine Rotationssymmetrie um die Längsachse der Sonde ist.

13. Verfahren zur endoskopischen nuklearen Bilderzeugung, umfassend:
- Einführen einer Nuklearsonde, die eine nicht-rotationssymmetrische Detektionscharakteristik aufweist, in den Körper eines Patienten;
- Fortlaufendes Aufnehmen von Strahlungsdaten mittels der Nuklearsonde;
- Fortlaufendes Aufnehmen von Positions- und Orientierungsdaten der Nuklearsonde,
- Fortlaufendes Berechnen eines dreidimensionalen Bildes unter Nutzung der vorgenannten Information;
- Fortlaufendes Berechnen mindestens eines Qualitätswertes für das errechnete dreidimensionale Bild.

14. Verfahren nach Anspruch 13, weiter umfassend:
Errechnen von mindestens einer Soll-Position und Soll-Orientierung des länglichen Körpers der Nuklearsonde unter Nutzung der oben angeführten Informationen, mit der Nebenbedingung, dass eine weitere Aufnahme radioaktiver Daten unter Vorgabe der neuen Sollwerte zu einer Verbesserung des mindestens einen Qualitätswertes führt, und optional weiter umfassend:
Aufnehmen von Positionsdaten des Körpers des Patienten.

15. Verfahren nach einem der Ansprüche 13 bis 14, ferner umfassend:
- Ausgeben einer Anweisung an einen Benutzer zum Bewegen des länglichen Körpers der endoskopischen Nuklearsonde an die Soll-Position und Soll-Orientierung;
- Aufnehmen zusätzlicher Daten mittels der Nuklearsonde mit den neuen Positionsdaten;
- erneutes Berechnen des dreidimensionalen Bilds und des Qualitätswerts,
- Ausgeben des Bilds auf einem Display.
